# EUROPÄISCHE PATENTANMELDUNG

(11) **EP 1 557 375 A1**
(43) Veröffentlichungstag der Anmeldung: **27.07.2005**
(21) Anmeldenummer: 04001429.2
(22) Anmeldetag: 23.01.2004
(51) Int. Cl.: B65D 83/14, A61K 7/04

(54) **Nageltinktur-Sprühdose**

(71) Anmelder: Neubourg Skin Care GmbH & Co. KG, 48282 Emsdetten (DE)
(72) Erfinder: Neubourg, Thomas, 59368 Werne (DE)
(74) Vertreter: Maiwald, Walter, Dr. Dipl.-Chem.

(57) **Zusammenfassung**

Eine Nageltinktur-Sprühdose weist einen Tinkturbehälter (10) auf, in dem eine Nageltinktur enthalten ist. Die Nageltinktur weist einen Wirkstoff, eine Pflegekomponente, eine Lösungsmittel und Treibgas auf. Der Tinkturbehälter (10) ist mit einem Deckel (12) verschlossen, wobei der Deckel (12) ein Auslassventil (16) trägt. Zur Betätigung des Auslassventils (16) ist auf den Deckel (12) ein Sprühkopf (20) aufgesteckt. In dem Sprühkopf (20) ist zur Durchleitung der Tinktur ein Auslasskanal (32) vorgesehen. Um die Nageltinktur aus einem größeren Abstand gezielt auf einen Nagel, insbesondere einen Fußnagel, aufbringen zu können, ist der Auslasskanal (32) erfindungsgemäß mit einem Rohransatz (34) verbunden.

## Beschreibung

Die Erfindung betrifft eine Nageltinktur-Sprühdose, mit der eine Nageltinktur auf einen mit einem Pilz oder dgl. befallenen Nagel aufgetragen werden kann. Ferner betrifft die Erfindung die Verwendung einer Sprühdose für Nageltinktur.

Zur Behandlung von beispielsweise einem mit Pilz o. ä. befallenen Nagel, insbesondere einem Fußnagel, ist es erforderlich, diesen mit einer Nageltinktur zu behandeln. Die Nageltinktur kann beispielsweise mit Hilfe eines Pinsels aufgetragen werden. Dies führt jedoch zur Kontaminierung des Pinsels und somit ggf. zur Übertragung des Erregers auf andere Nägel. Ferner kann die Nageltinktur mit einer Pipette aufgetragen werden, wobei auch hierbei die Gefahr besteht, dass die Pipettenspitze kontaminiert wird. Ferner weist das Aufbringen der Nageltinktur mit einem Pinsel oder einer Pipette den Nachteil auf, dass dies insbesondere für ältere Patienten schwierig ist, da zum Aufbringen der Nageltinktur mit Hilfe eines Pinsels oder einer Pipette eine gute Beweglichkeit des Patienten gewährleistet werden muss, damit der Patient die Tinktur selbst aufbringen kann.

Eine weitere Möglichkeit zum Aufbringen der Nageltinktur besteht darin, die Nageltinktur mit einer Sprühdose auf den Nagel aufzusprühen. Dies hat jedoch den Nachteil, dass die Nageltinktur zerstäubt wird und es daher für ein gezieltes Aufbringen der Nageltinktur auf die befallene Stelle erforderlich ist, den Sprühkopf sehr nahe an den Nagel heranzuführen. Dies hat wiederum den Nachteil, dass dies für insbesondere ältere Patienten schwierig ist.

Aufgabe der Erfindung ist es, eine Nageltinktur-Sprühdose zu schaffen, mit der das Auftragen von Nageltinktur auf Fußnägel für insbesondere ältere Patienten einfach durchführbar ist.

Die Lösung der Aufgabe erfolgt erfindungsgemäß durch eine Nageltinktur-Sprühdose gemäß Anspruch 1 sowie durch die Verwendung einer Sprühdose zum Aufbringen von Nageltinktur gemäß Anspruch 12.

Die Nageltinktur-Sprühdose weist einen Tinkturbehälter auf, in dem die Nageltinktur enthalten ist. Die Nageltinktur weist einen Wirkstoff, eine Pflegekomponente, ein Lösungsmittel und ein Treibgas auf. Der Tinkturbehälter, der üblicherweise aus Metall ist, ist mit einem Deckel dicht verschlossen. Der Deckel weist beispielsweise einen metallenen Rand auf oder ist vollständig aus Metall, wobei ein dichtes Verbinden des Tinkturbehälters mit dem Deckel durch Umbördeln realisiert werden kann. In dem Deckel ist ein Auslassventil angeordnet. Bei dem Auslassventil kann es sich um ein Röhrchen handeln, wobei durch geringfügiges Eindrücken in den Tinkturbehälter eine Auslassöffnung des Tinkturbehälters geöffnet wird, so dass durch diese die Tinktur ausströmen kann. Des weiteren weist die Nageltinktur-Sprühdose einen auf den Deckel bzw. das Auslassventil aufgesetzten Sprühkopf zum Betätigen des Auslassventils auf. Durch den Sprühkopf verläuft ein mit dem Auslassventil, insbesondere dem Röhrchen des Auslassventils, verbundener Auslasskanal, durch den beim Betätigen des Sprühkopfes die Tinktur zu einer Auslassöffnung strömt und an dieser versprüht wird.

Um ein gezieltes Aufbringen der Nageltinktur auf einen Fußnagel auch aus einem größeren Abstand zu ermöglichen, ist der Auslasskanal des Sprühkopfes mit einem Rohransatz verbunden. Die Tinktur tritt somit nicht aus dem Sprühkopf aus und wird hierbei zerstäubt, sondern wird durch den Rohransatz gebündelt, so dass im Wesentlichen ein Strahl entsteht, der sodann von dem Patienten auf den befallenen Nagel gerichtet werden kann. Es ist somit möglich, auch in Abständen, die mehr als 10 cm betragen können, gezielt Nageltinktur auf einen befallenen Fußnagel aufzubringen. Das Aufbringen von Nageltinktur auf einen Fußnagel ist durch Verwenden der erfindungsgemäßen Nageltinktur-Sprühdose insbesondere für ältere Patienten erheblich vereinfacht. Ferner ist eine Kontamination ausgeschlossen.

An Stelle einer Nageltinktur kann mit der erfindungsgemäßen Sprühdose auch eine andere Tinktur oder anderer Wirkstoff gezielt aus einem größeren Abstand auf eine schwer erreichbare Körperstelle aufgetragen werden. Die Erfindung ist somit nicht auf den Einsatz von Nageltinktur beschränkt, jedoch hierfür besonders geeignet.

Vorzugsweise ist der Rohransatz fest mit dem Sprühkopf verbunden. Insbesondere ist der Rohransatz und der Sprühkopf einstückig. Hierbei kann es sich um ein, beispielsweise durch Spritzgießen hergestelltes, Kunststoffteil handeln. Der Rohransatz weist vorzugsweise eine Länge von mehr als 10 mm und besonders bevorzugt mehr als 15 mm auf. Ferner ist es möglich, den Rohransatz vor dem Benutzen der Nageltinktur-Sprühdose beispielsweise auf einen Ansatzstutzen des Sprühkopfes zum Verbinden des Rohransatzes mit dem Auslasskanal aufzustecken.

Bei einer weiteren bevorzugten Ausführungsform ist der Rohransatz zumindest teilweise von einer Versteifungshülse umgebeben. Bei einem zylindrischen Rohransatz handelt es sich bei der Versteifungshülse um einen geschlossenen Hohlzylinder, der beispielsweise über Stege mit dem Rohransatz verbunden ist. Vorzugsweise sind mehrere Versteifungshülsen vorgesehen, die insbesondere konzentrisch zueinander angeordnet sind. Hierdurch ist ein Verformen des Rohransatzes Vermieden und die Herstellung vereinfacht, wobei die Versteifungshülsen vorzugsweise mit dem Rohransatz und/ oder dem Sprühkopf verbunden sind. Insbesondere handelt es sich bei dem Rohransatz und den Versteifungshülsen um ein einstückiges Teil, das vorzugsweise aus Kunststoff hergestellt ist.

Vorzugsweise ist die mindestens eine Versteifungshülse mit dem Sprühkopf verbunden und vorzugsweise einstückig, insbesondere aus Kunststoff hergestellt. Hierbei ist die Versteifungshülse vorzugsweise derart mit dem Sprühkopf verbunden, dass der Rohransatz auf der dem Sprühkopf abgewandten Seite über die Versteifungshülse vorsteht.

Der vorzugsweise in dem Tinkturbehälter vorgesehene Wirkstoff ist insbesondere ein Antimykotikum wie Clotrimazol und/ oder ein antimikrobieller Wirkstoff wie Undecylenamid DEA. Als Pflegekomponenten kommen insbesondere solche in Betracht, die pflanzlichen Ursprungs sind, wie Persea gratissima. Auch andere an sich bekannte hautpflegende Mittel wie Panthenol sind einsetzbar. Gegebenenfalls können weitere Hilfsstoffe zur Herstellung einer Tinktur oder zur Konservierung zugefügt.

Erfindungsgemäß können in der Tinktur auch zusätzlich Lösungsvermittler wie Popylenglykol vorhanden sein.

Typischerweise ist das Lösungsmittel in der Nageltinktur eine wässrige Lösung eines hautverträglichen Alkanols oder das hautverträgliche Alkanol selbst. Vorzugsweiae wird als hautverträgliches Alkanol Ethanol eingesetzt.

Als Treibgas kommen Propan und/ oder Butan in Betracht.

Die einzelnen Komponenten können in folgenden Mengenbereichen (Gew %) vorliegen:

| | | |
|---|---|---|
| Lösungsmittel | 60 - 80 %, insbesondere | 65 - 75 % |
| Pflegekomponente | 0,1 - 10 %, insbesondere | 0,5- 4.% |
| Wirkstoff | 0,1 - 10 %, insbesondere | 0,5 - 2 % |
| Treibgas | 15 - 35 %, insbesondere | 20 - 30 |

Ferner betrifft die Erfindung die Verwendung der vorstehend beschriebenen Sprühdose für die vorstehend beschriebene Nageltinktur.

Nachfolgend wird die Erfindung anhand einer bevorzugten Ausführungsform unter Bezugnahme auf die anliegenden Zeichnungen näher erläutert.

Es zeigen:
- Figur 1: eine schematische Seitenansicht der Nageltinktur-Sprühdose,
- Figur 2: einen schematischen Längsschnitt des Sprühkopfes und
- Figur 3: eine schematische Draufsicht des Sprühkopfes.

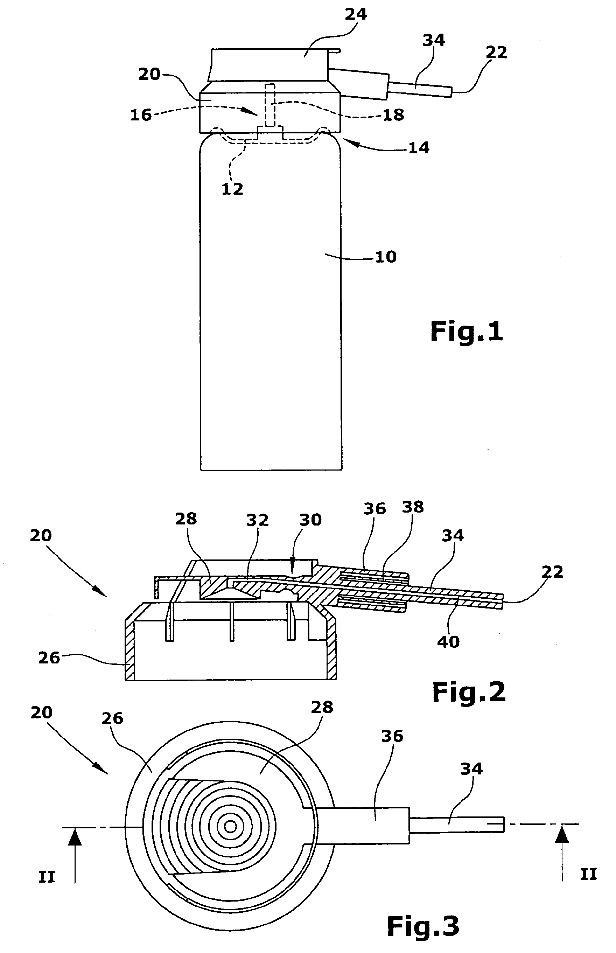

Die erfindungsgemäße Nageltinktur-Sprühdose weist einen Tinkturbehälter 10 auf, in dem eine Nageltinktur enthalten ist. Die Nageltinktur weist einen Wirkstoff, eine Pflegekomponente, ein Lösungsmittel und ein Treibgas auf. Der Tinkturbehälter 10, der üblicherweise aus Metall ist, steht unter Druck und ist mit einem Deckel 12 verschlossen. Der Deckel 12 ist üblicherweise ebenfalls aus Metall und an seinem Rand 14 durch Umbördeln mit dem im Wesentlichen zylindrischen Tinkturbehälter 10 verbunden. Innerhalb des Deckels 12 ist ein Auslassventil 16 mit einem rohrförmigen Auslass 18 angeordnet. Durch Drücken des rohrförmigen Auslasses 18 in Figur 1 nach unten wird das Ventil geöffnet, so dass aus dem Tinkturbehälter 10 aufgrund des Innendrucks Tinktur durch das Auslassröhrchen 18 nach oben strömt.

Die durch das Auslassröhrchen 18 strömende Tinktur wird durch einen Sprühkopf 20 zu einem Auslass 22 geleitet. Der Sprühkopf 20 ist mit einem Deckel 24 verschlossen, um ein ungewolltes Betätigen des Sprühkopfes zu vermeiden.

Der Sprühkopf 20 (Fig. 2) weist einen im Wesentlichen zylindrischen Körper 26 auf, der auf den Deckel 12 aufgesteckt ist. Ferner weist der Sprühkopf 20 ein Betätigungsteil 28 auf, das über eine Verjüngung 30 flexibel mit dem Körper 26 des Sprühkopfes 20 verbunden ist. Das Betätigungselement 28 kann somit in Figur 2 nach unten gedrückt werden und gelangt aufgrund der elastischen Verbindung 30 nach dem Loslassen wieder in die in Figur 2 dargestellte Ausgangsstellung zurück. Durch Betätigen des Betätigungselementes 28 wird das Auslassröhrchen 18 des Ventils 16 in Figur 1 nach unten gedrückt und somit das Ventil geöffnet. Die ausströmende Tinktur gelangt hierbei in einem in dem Sprühkopf 20 vorgesehenen Auslasskanal 32 und strömt durch diesen in Richtung der Auslassöffnung 22.

Der Auslasskanal 32 erstreckt sich innerhalb des Betätigungselementes 28, indem er zur Seite abgewinkelt ausgebildet ist. Der Auslasskanal mündet sodann in einen Rohransatz 34, der vorzugsweise einstückig mit dem Sprühkopf 20 verbunden ist.

Zur Versteifung des Rohransatzes 34 ist dieser teilweise von einer Versteifungshülse 36 und einer weiteren konzentrisch zur Versteifungshülse 36 angeordneten Versteifungshülse 38 umgeben. Zwischen den Versteifungshülsen 36, 38 und dem Rohransatz 34 sind Stege vorgesehen, durch die die Teile miteinander verbunden sind.

Durch Betätigen des Betätigungselementes 28 wird somit das Ventil 16 geöffnet und die Tinktur strömt durch den Auslasskanal 32 in Richtung der Auslassöffnung 22. Aufgrund des erfindungsgemäßen Vorsehens eines Rohransatzes 34 mit einem dünnen Kanal 40, in den der Auslasskanal 32 mündet, ist gewährleistet, dass die Tinktur aus der Auslassöffnung 22 aus einer größeren Entfernung gezielt auf einen zu behandelnden Nagel oder dgl. abgegeben werden kann.

Die Nageltinktur weist folgende zusammensetzungen auf (%):

| | |
|---|---|
| Alcohol denat. | 70,83 |
| Butan | 25,00 |
| Persea gratissima | 1,51 |
| Clotrimazol | 1,01 |
| Panthenol | 0,76 |
| Undecylenamid | 0,15 |
| Propylenglykol | 0,74 |

## Patentansprüche

1. Nageltinktur-Sprühdose, mit
einem Tinkturbehälter (10), in dem Nageltinktur mit einem Wirkstoff, einer Pflegekomponente, einem Lösungsmittel und einem Treibgas enthalten ist,
einem den Tinkturbehälter (10) dicht verschließenden Deckel (12),
einem in dem Deckel (12) angeordnetem Auslassventil (16),
einem Sprühkopf (20) zum Betätigen des Auslassventils (16) und
einem durch den Sprühkopf (20) verlaufenden Auslasskanal (32)
**dadurch gekennzeichnet, dass**
der Auslasskanal (32) mit einem Rohransatz (34) zum gezielten Abgeben der Tinktur verbunden ist.

2. Nageltinktur-Sprühdose nach Anspruch 1, **dadurch gekennzeichnet, dass** der Rohransatz (34) fest mit dem Sprühkopf (20) insbesondere einstückig verbunden ist.

3. Nageltinktur-Sprühdose nach Anspruch 1 oder 2, **dadurch gekennzeichnet, dass** der Rohransatz (34) teilweise von einer Versteifungshülse (36, 38) umgeben ist.

4. Nageltinktur-Sprühdose nach Anspruch 3, **dadurch gekennzeichnet, dass** die Versteifungshülse (36, 38) mit dem Sprühkopf (20) verbunden ist, so dass der Rohransatz (34) auf der in dem Sprühkopf (20) abgewandten Seite über die Versteifungshülse (36, 38) vorsteht.

5. Nageltinktur-Sprühdose nach einem der Ansprüche 1 - 4, **dadurch gekennzeichnet, dass** der Wirkstoff ein Antimykotikum ist.

6. Nageltinktur-Sprühdose nach Anspruch 5, **dadurch gekennzeichnet dass** das Antibiotikum Clotrimazol ist.

7. Nageltinktur-Sprühdose nach einem der Ansprüche 1 - 6, **dadurch gekennzeichnet, dass** die Pflegekomponente pflanzlichen Ursprungs, wie Persea gratissima; Panthenol und/oder Undecylenamid DEA ist.

8. Nageltinktur-Sprühdose nach einem der Ansprüche 1 - 7, **dadurch gekennzeichnet, dass** zusätzlich Lösungsvermittler wie Popylenglykol in der Nageltinktur vorhanden ist.

9. Nageltinktur-Sprühdose nach einem der Ansprüche 1 - 8, **dadurch gekennzeichnet, dass** das Lösungsmittel eine wässrige Lösung eines hautverträglichen Alkanols oder ein hautverträgliches Alkanol ist.

10. Nageltinktur-Sprühdose nach einem der Ansprüche 1 - 9, **dadurch gekennzeichnet, dass** das hautverträgliche Alkanol Ethanol ist.

11. Nageltinktur-Sprühdose nach einem der Ansprüche 1 - 10, **dadurch gekennzeichnet, dass** das Treibgas Propan und/oder Butan ist.

12. Verwendung einer Sprühdose mit
einem mit einem Deckel (12) dicht verschlossenen Behälter (10),
einem in dem Deckel (12) angeordnetem Auslassventil (16),
einem Sprühkopf (20) zum Betätigen des Auslassventils (16),
einem durch den Sprühkopf (20) verlaufenden Auslasskanal (32) und
einem mit dem Auslasskanal (32) verbundenen Rohransatz (34)
für die gezielte Abgabe von in dem Behälter (10) enthaltener Nageltinktur mit einem Wirkstoff, einer Pflegekomponente, einem Lösungsmittel und einem Treibgas.
